# EUROPEAN PATENT APPLICATION

(11) **EP 4 786 569 A1**
(43) Date of publication of application: **05.08.2026**
(21) Application number: 24871159.0
(22) Date of filing: 26.09.2024
(51) Int. Cl.: C12M 1/00, C12M 1/28

(54) **LID, VESSEL FOR CELL CULTURE DEVICE OR BIOREACTOR, AND CELL CULTURE DEVICE**

(30) Priority: 29.09.2023 JP 2023169329
(71) Applicant: ZACROS Corporation, Tokyo 112-0002 (JP)
(72) Inventor: MATSUDA, Wakana, Tokyo 112-0002 (JP); MATSUDA, Hiroyuki, Tokyo 112-0002 (JP)
(74) Representative: Godemeyer Blum Lenze Patentanwälte Partnerschaft mbB - werkpatent
(86) International application number: PCT/IB2024/059389
(87) International publication number: WO 2025/068929

(57) **Abstract**

The present invention provides a lid by which a tube can be easily handled and positioned and which has a low risk of ingress of foreign matters from an end portion of the tube, a vessel for a cell culture device or a bioreactor including the lid, and a cell culture device for culturing a cell using the vessel. More specifically, the present invention provides a lid 2 for occluding an opening end of a vessel body 4, the lid 2 including: a lid body 6 made of a heat insulating material; a flexible member 8 for occluding an insertion portion 7 formed in the lid body 6; and an insertion slit 18 that can be elastically widened formed in the flexible member 8. The flexible member 8 is formed of a flexible material having higher flexibility than that of the heat insulating material.

## Description

### TECHNICAL FIELD

The present invention relates to a lid for occluding an open end of a vessel body and inserting a tube and the like from outside of the vessel body into the vessel body and fixing the tube and the like, a vessel for a cell culture device or a bioreactor including the above-mentioned lid, and a cell culture device for culturing a cell using the above-mentioned vessel.

### BACKGROUND TECHNOLOGY

As this type of cell culture device or bioreactor, for example, a shake-type culture device shown in Patent Document 1 is known. The shake-type culture device disclosed in Patent Document 1 includes a culture bag enclosing a liquid, an outer shell vessel for housing the above-mentioned culture bag, and a shaking mechanism for shaking the above-mentioned outer shell vessel to stir the liquid, and is used, for example, when a microorganism or a cell is cultured in the above-mentioned liquid, or the like. The above-mentioned outer shell vessel includes a bottomed cylindrical vessel body, and is used by housing a culture bag enclosing the above-mentioned liquid in this vessel body, and occluding an opening end of the above-mentioned vessel body with a plate-like lid so as to fix the above-mentioned culture bag in the above-mentioned vessel body.

### PRIOR ART DOCUMENT

### Patent Document

Patent Document 1: Japanese Unexamined Patent Application, First Publication No. 2016-136928

### SUMMARY OF THE INVENTION

### PROBLEMS TO BE SOLVED BY THE INVENTION

Incidentally, the temperature is optimized for the activity of a cell, an enzyme, and the like in a cell culture vessel or a bioreactor, and a temperature difference generally occurs between the inside and the outside of the cell culture vessel or the bioreactor. However, a large number of tubes and the like are connected to the upper portion of the cell culture vessel or the bioreactor, and thus there has been a problem that it is difficult to control the temperature in the cell culture vessel or the bioreactor.

The present invention has been made in view of the above-mentioned circumstances, and an objective of the present invention is to provide a lid by which a tube can be easily handled and positioned and which has a low risk of ingress of foreign matters from an end portion of the tube, a vessel for a cell culture device or a bioreactor including the lid, and a cell culture device for culturing a cell using the vessel.

### MEANS FOR SOLVING THE PROBLEM

A first aspect of the present invention is a lid for occluding an open end of a vessel body for a cell culture vessel or a bioreactor, wherein the lid includes a lid body having a heat insulation function, an insertion portion communicating from a front surface to a back surface of the lid body is formed in the lid body, and the insertion portion is provided with a flexible member having an insertion slit that can be elastically widened.

According to the first aspect, when a tube, a stirring rod, and the like (hereinafter referred to as "tube and the like") are disposed through the lid, the insertion slit can be widened to insert the tube and the like from a side surface of the lid, the tube and the like can be moved to an appropriate position along the insertion slit, and the tube and the like after the movement can be held by an elastic force of the insertion slit. Therefore, the first aspect exerts excellent effects that the tube and the like can be easily handled and positioned, and that a risk of ingress of foreign matters from an end portion of the tube is low.

In a second aspect of the present invention, the lid body is allowed to be divided into two or more parts with a division plane as a boundary in the first aspect.

According to the second aspect, the insertion slit can be widened to insert the tube and the like from the division plane, and the tube and the like can be moved to an appropriate position along the insertion slit, in a state where the lid body is divided into two or more with the division plane as a boundary. Therefore, the second aspect exerts an effect that the tube and the like can be easily handled and positioned.

In a third aspect of the present invention, in the first or second aspect, the insertion portion extends to an outer peripheral edge of the lid body or the division plane formed in the lid body.

According to the third aspect, one end of the insertion slit that extends to the outer peripheral edge or the division plane of the lid body can be widened to insert the tube and the like from a side surface. Therefore, the third aspect exerts an effect that the tube and the like can be easily handled and positioned.

In a fourth aspect of the present invention, in any of the first to third aspects, the insertion portion and the flexible member have an identical shape, and the flexible member is embedded in the insertion portion.

According to the fourth aspect, an effect is exerted that the insertion portion can be formed without significantly increasing the thickness of the lid.

In a fifth aspect of the present invention, in any of the first to fourth aspects, a first insertion slit that extends to the outer peripheral edge and/or the division plane of the lid body, and a second insertion slit branching from the first insertion slit are formed as the insertion slit in the insertion portion.

According to the fifth aspect, an end portion of the first insertion slit that extends to the outer peripheral edge and/or the division plane of the lid body can be widened to insert the tube and the like from a side surface, and then the tube and the like can be moved to an appropriate position along the second insertion slit branching from the first insertion slit. Therefore, the fifth aspect exerts effects that the degree of freedom of holding positions for the tube and the like is high, and that the tube and the like can be easily handled and positioned.

In a sixth aspect of the present invention, in any of the first to fifth aspects, the insertion portion has a wide portion formed to be wider than other portions of the insertion portion in plan view. Furthermore, since the wide portion has a wide width, a plurality of insertion slits may be formed.

According to the sixth aspect, the tube and the like can be inserted from a side surface along the insertion slit in the narrow portion of the insertion portion, and then the tube and the like can be moved by selecting either of the insertion slits in the wide portion. Therefore, the sixth aspect exerts effects that the degree of freedom of holding positions for the tube and the like is high, and that the tube and the like can be easily handled and positioned.

In a seventh aspect of the present invention, in the sixth aspect, the wide portion has a substantially circular shape in plan view. In the wide portion in this case, for example, a plurality of insertion slits may be formed radially from a central portion of the substantially circular portion.

According to the seventh aspect, the tube and the like can be inserted from a side surface along the insertion slit in the narrow portion of the insertion portion, and then the tube and the like can be moved by selecting either of the plurality of insertion slits radially formed in the wide portion. Therefore, the seventh aspect exerts effects that the degree of freedom of holding positions for the tube and the like is high, and that the tube and the like can be easily handled and positioned.

An eighth aspect of the present invention is a vessel for a cell culture device or a bioreactor, including: a vessel body having an open end; and the lid according to any one of the first to seventh aspects.

According to the eighth aspect, when the tube, the stirring rod, and the like that extend from outside into the vessel body are disposed through the lid, the insertion slit can be widened to insert the tube and the like from a side surface, the tube and the like can be moved to an appropriate position along the insertion slit, and the tube and the like after the movement can be held by an elastic force of the insertion slit. Therefore, the eighth aspect exerts effects that the tube and the like can be easily handled and positioned, and that a risk of contamination of an end of the tube and the like is low.

In a ninth aspect of the present invention, in the eighth aspect, a fixing portion for fixing the lid and the vessel body to each other is formed on the lid and the vessel body.

According to the ninth aspect, since the lid can be fixed in a state of being fitted to the open end of the vessel body by the fixing portion, the lid can be prevented from coming off also in the case when the vessel body is shaken.

A tenth aspect of the present invention includes an inner bag in the vessel body in the eighth or ninth aspect.

According to the tenth aspect, cell culture or various reactions can be performed in the inner bag in a state where heat insulation and airtightness are enhanced by inserting the tube, the stirring rod, and the like into the inner bag through the insertion slit of the lid.

An eleventh aspect of the present invention is a cell culture device including: the vessel for a cell culture device or a bioreactor according to any one of the eighth to tenth aspects; a shaking mechanism for shaking the vessel; a temperature adjustment mechanism for adjusting the temperature of the vessel; and a tube to be inserted into the vessel through the insertion slit of the lid.

According to the eleventh aspect, in a case where a liquid to be treated is housed directly in the vessel body or through a packaging bag, and the tube and the like that extend into the vessel body from outside are disposed through the lid, the insertion slit can be widened to insert the tube and the like from a side surface, the tube and the like can be moved to an appropriate position along the insertion slit, and the tube and the like after the movement can be held by an elastic force of the insertion slit. Therefore, the tube and the like can be easily handled, and a risk of contamination of an end of the tube is low, and in a case where the vessel is shaken by the shaking mechanism while the temperature of the vessel is adjusted by the temperature adjustment mechanism, a heat retention effect by the lid can also be obtained.

### EFFECTS OF INVENTION

As described above, according to the lid, the vessel for a cell culture device or a bioreactor, and the cell culture device of the present invention, when the tube and the like are disposed through the lid, the insertion slit can be widened to insert the tube and the like from a side surface, the tube and the like can be moved to an appropriate position along the insertion slit, and the tube and the like after the movement can be held by an elastic force of the insertion slit. Therefore, the tube and the like can be easily handled and positioned, and a risk of ingress of foreign matters from an end of the tube is low.

### BRIEF DESCRIPTION OF DRAWINGS

Fig. 1 is a plan view of the lid according to the first embodiment of the present invention.

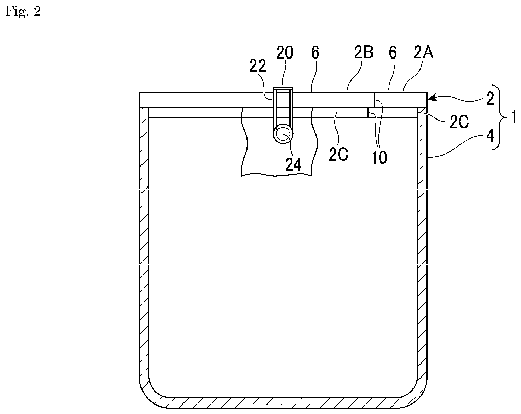

Fig. 2 is a partially cutaway front view of the vessel for a cell culture device or a bioreactor using the lid of the first embodiment.
Fig. 3 is an enlarged cross-sectional view showing an example of the division plane in the lid of the first embodiment.
Fig. 4 is an enlarged cross-sectional view showing another example of the division plane in the lid of the first embodiment.
Fig. 5 is an enlarged cross-sectional view showing another example of the division plane in the lid of the first embodiment.
Fig. 6 is an enlarged cross-sectional view illustrating the lid body, the insertion portion, and the insertion slit in the lid of the first embodiment.
Fig. 7 is an enlarged cross-sectional view illustrating a modified example of the lid body, the insertion portion, and the insertion slit in the lid of the first embodiment.
Fig. 8 is an enlarged cross-sectional view illustrating a modified example of the lid body, the insertion portion, and the insertion slit in the lid of the first embodiment.
Fig. 9 is a plan view illustrating the first portion in the case where the lid of the first embodiment is divided into two from the division plane.
Fig. 10 is a plan view illustrating the second portion in the case where the lid of the first embodiment is divided into two from the division plane.
Fig. 11 is a plan view illustrating the state in which a plurality of tubes are inserted and held in the insertion portions of the lid of the first embodiment.
Fig. 12 is a partially cutaway front view of a vessel illustrating the state in which a plurality of tubes are inserted and held in the insertion portions of the lid of the first embodiment.
Fig. 13 is a partially cutaway front view of a cell culture device including the vessel using the lid of the first embodiment.
Fig. 14 is a plan view of the lid according to the second embodiment of the present invention.
Fig. 15 is a plan view of the lid according to the third embodiment of the present invention.
Fig. 16 is a plan view of the lid according to the fourth embodiment of the present invention.
Fig. 17 is a plan view of the lid according to the fifth embodiment of the present invention.

### BEST MODE FOR CARRYING OUT THE INVENTION

Hereinafter, a plurality of embodiments of the present invention will be described in detail with reference to the drawings.

### [First Embodiment]

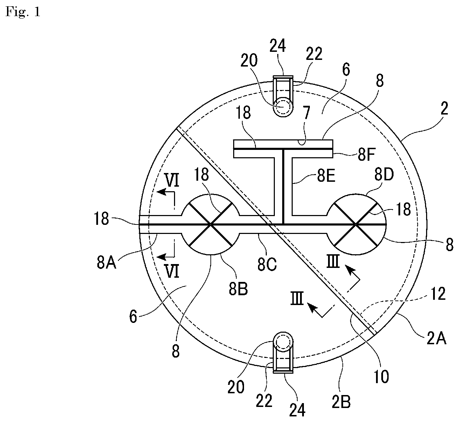

Fig. 1 is a plan view of a lid 2 according to the first embodiment of the present invention. The lid 2 is used by being fitted to an open end of a vessel body 4 for occluding the open end, and a vessel 1 is configured by the lid 2 and the vessel body 4. The vessel 1 is used as a cell culture device or a bioreactor. The lid 2 forms a disk shape having substantially an identical diameter with the outer diameter of the vessel body 4, and the vessel body 4 forms a bottomed cylindrical shape. Although the size of the vessel 1 is not particularly limited, for example, the vessel body 4 may have an outer diameter of 10 to 500 cm and a height of 20 to 500 cm. The vessel body 4 may have a polygonal columnar shape such as a rectangular parallelepiped shape, and in that case, the shape of the lid 2 is also matched with the shape of the open end of the vessel body 4.

The lid 2 is formed of a disk-shaped lid body 6 and a flexible member 8 embedded and fixed in an insertion portion 7, which is a notch portion or an opening portion formed in this lid body 6. The lid body 6 is preferably formed of a material having an excellent heat insulation property, and the flexible member 8 is formed of a flexible material having more flexibility than that of the material of the lid body 6. The flexible member 8 and the insertion portion 7 have an identical shape, and the flexible member 8 is fitted into the insertion portion 7 without any gap and is joined by an adhesive or thermal fusion bonding. The material having an excellent heat insulating property is not particularly limited, and for example, expanded polystyrene, expanded polyethylene, expanded polypropylene, other expanded resins, and the like are preferable because of their high heat insulating properties and light weights. As the flexible material, soft urethane foams, urethane rubbers, silicone rubbers, fluoro rubbers, natural rubbers, synthetic rubbers, and the like can be used. The flexible material may be inferior in heat insulating property to the above-mentioned heat insulating material.

In the flexible member 8 of this embodiment, a band-shaped linear portion 8A having one end that extends to the outer periphery of the lid body 6, a disk-shaped circular portion 8B (wide portion), a band-shaped linear portion 8C, and a disk-shaped circular portion 8D (wide portion) are formed side by side and connected to each other in a row, and further, a band-shaped linear portion 8E is formed by vertically branching from the center of the linear portion 8C, and a T-shaped portion 8F (wide portion) having a constant width forming a T-shape is formed at an end of the linear portion 8E. The flexible member 8 is formed with linear insertion slits 18 that are vertical cuts along the center lines of the linear portion 8A, the circular portion 8B, the linear portion 8C, the circular portion 8D, the linear portion 8E, and further, the T-shaped portion 8F, and the circular portions 8B and 8D are formed with a plurality of (four in the illustrated example) insertion slits 18 radially from the centers of the circles.

The insertion slit 18 is preferably formed perpendicular to the upper surface of the lid 2, but may be slightly inclined. The insertion slit 18 is made elastically widenable to such an extent that a tube 32 and the like described later can be inserted by a flexible material constituting the flexible member 8. In the case where the tube 32 and the like are not inserted into the insertion slit 18, it is preferable that the insertion slit 18 is closed in order to enhance the heat insulating property of the lid 2. However, a slight gap may be opened since the influence on the heat insulating property is small.

The shape of the flexible member 8 in Fig. 1 is an example, and the shape and arrangement may be arbitrarily changed as necessary. The thickness of the flexible member 8 is not particularly limited, but in this embodiment, the thickness of the flexible member 8 is identical with that of the lid body 6, and the upper and lower surfaces of the lid body 6 and the upper and lower surfaces of the flexible member 8 are flush with each other. On the lower surface of the outer peripheral portion of the lid 2, a step-shaped engagement portion 2C that is cut out at a constant width over the entire circumference is formed so that the upper end portion of the vessel body 4 fits in an airtight manner. The thickness of the lid 2 is not particularly limited, but may be, for example, 2 to 20 cm in order to obtain sufficient heat insulating property and strength.

On the upper surface of the lid 2, two fasteners 20 are attached at positions separated by 180°. The number of the fasteners is not limited to two, and three or more fasteners may be provided at equal intervals or unequal intervals in the circumferential direction. The fastener 20 of this example has a column portion protruding from the surface of the lid body 6 and a disk portion formed at the tip of the column portion, and fixes one end of an elastically stretchable ring 22 by hooking the ring 22 over the disk portion. Similar fasteners 24 are fixed to the upper end portion of the outer peripheral surface of the vessel body 4 at two positions corresponding to the respective fasteners 20, and the lid 2 is detachably fixed to the vessel body 4 by respectively hooking the rings 22 between the fasteners 20 and the corresponding fasteners 24. The fixing structure between the lid 2 and the vessel body 4 is not limited to the ring 22, and may be another engagement structure.

A division plane 10 is formed on the lid 2 by passing through a position close to the center of the lid 2, and the lid 2 is made dividable into two: a semicircular first portion 2A and a semicircular second portion 2B. The division plane 10 divides the linear portion 8C, and the linear portion 8C and the insertion slit 18 are exposed at the division plane 10. As a result, the tube 32 and the like can also be inserted through the insertion slit 18 exposed on the division plane 10. Two or more division planes 10 may be formed.

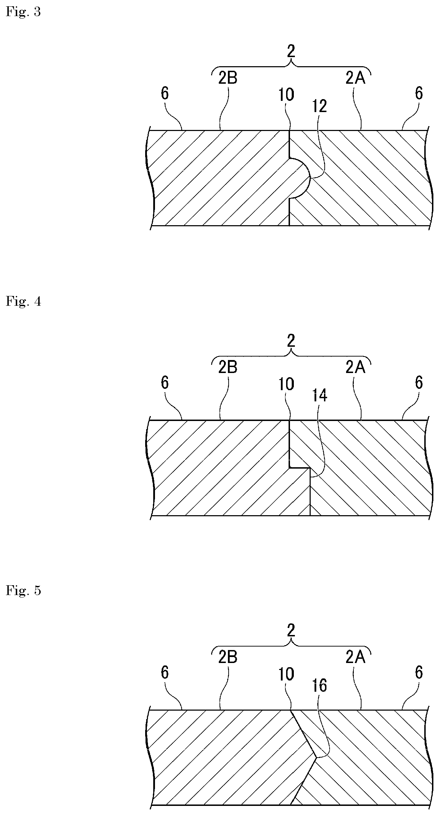

Figs. 3 to 5 illustrate examples of the cross-sectional shape of the division plane 10.

In the example of Fig. 3, an engaging portion 12 having a semicircular cross section and an engaging groove are formed in the central portion of the division plane 10, and the engaging portion 12 is fitted into the engaging groove, so that it is possible to prevent displacement of the first portion 2A and the second portion 2B in the vertical direction.

In the example of Fig. 4, a step-shaped engaging portion 14 and a stepped portion forming a complementary shape are formed at the central portion of the division plane 10, and the engaging portion 14 is fitted into the stepped portion, whereby the first portion 2A and the second portion 2B can be positioned in the vertical direction.

In the example of Fig. 5, a V-shaped engaging portion 16 and a V-shaped groove having a complementary shape are formed on the division plane 10, and the engaging portion 16 is fitted into the V-shaped groove, whereby the first portion 2A and the second portion 2B can be positioned in the vertical direction.

These cross-sectional shapes are merely examples, and are not limited to these, and it is also possible to form the division plane 10 simply perpendicular to the lid body 6, in which the engaging portion as described above is not formed.

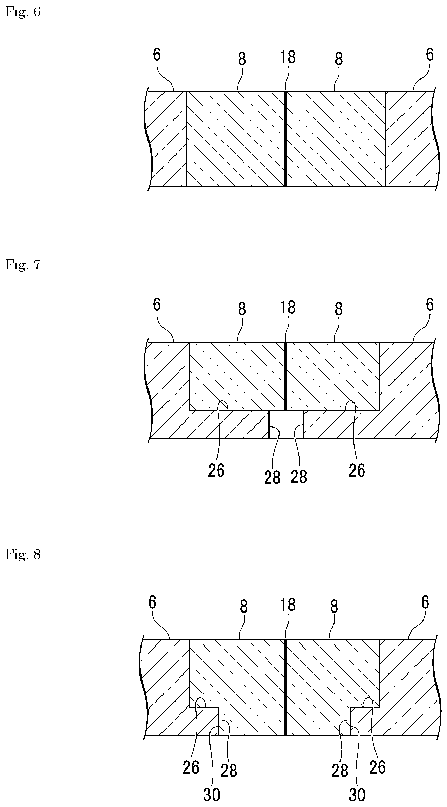

Figs. 6 to 8 illustrate examples of the cross-sectional shape of a joint portion between the lid body 6 and the flexible member 8.

In the example of Fig. 6, the thickness of the flexible member 8 is identical with that of the lid body 6, and both are joined so as to be flush with each other in the upper and lower surfaces.

In the example of Fig. 7, the thickness of the flexible member 8 is smaller than that of the lid body 6, an engagement portion 26 protruding toward the flexible member 8 in a stepwise manner is formed in the lower portion of the lid body 6, a slit 28 having a constant width is formed between the engagement portions 26, and an insertion slit 18 is formed in the flexible member 8 corresponding to the slit 28. In this example, since the upper surface of the engaging portion 26 can also be joined to the flexible member 8, a fixing force between the lid body 6 and the flexible member 8 can be enhanced. In addition, since the flexible member 8 becomes thin, the force required for widening the insertion slit 18 can be reduced.

In the example of Fig. 8, the thickness of the flexible member 8 is identical with that of the lid body 6, an engaging portion 26 protruding toward the flexible member 8 in a stepwise manner is formed in the lower portion of the lid body 6, a slit 28 having a constant width is formed between the engaging portions 26, and a ridge portion 30 that fits to the slit 28 is formed in the flexible member 8. An insertion slit 18 is formed in the flexible member 8 so as to pass through the central portion of the ridge portion 30. In this example, since the upper surface and the tip end surface of the engaging portion 26 can also be joined to the ridge portion 30 of the flexible member 8, the fixing force between the lid body 6 and the flexible member 8 can be enhanced.

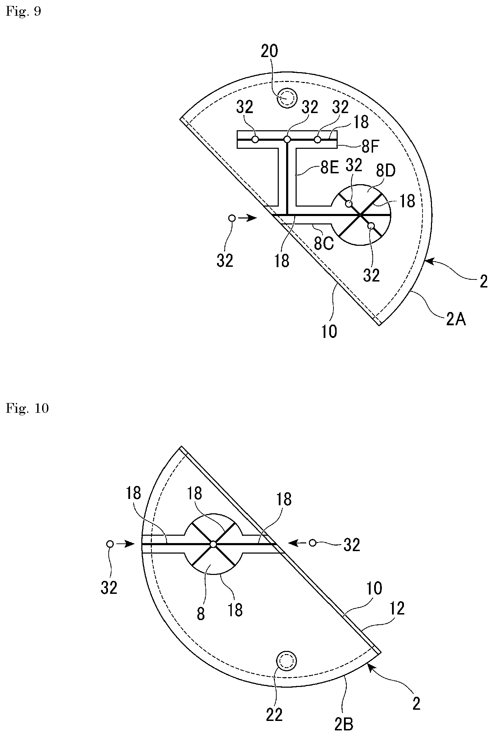

Figs. 9 and 10 illustrate the first portion 2A and the second portion 2B divided with the division plane 10 as a boundary. In this embodiment, by dividing the lid 2 by the division plane 10, the tube 32 and the like can be inserted into the insertion slit 18 also from a cross section of the insertion slit 18 exposed on the division plane 10, as indicated by arrows in these drawings. Therefore, as compared with the case of non-division in which the insertion slit 18 is opened only on the outer peripheral portion of the lid 2, it is easy to insert a large number of tubes 32 and the like into the insertion slit 18, and it is possible to arrange the tubes 32 and the like at desired holding positions in a shorter time.

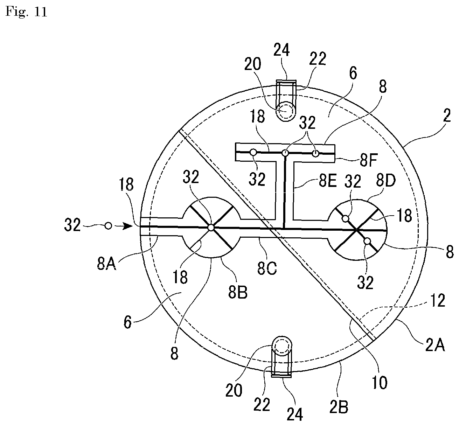

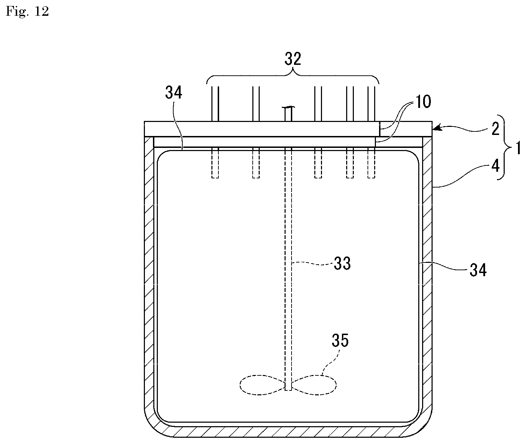

Figs. 11 and 12 are a plan view and a partially cutaway front view illustrating the first portion 2A and the second portion 2B illustrated in Figs. 9 and 10 showing the state in which the division plane 10 is closed in the state that the engaging portions 12 are fitted together, and the first portion 2A and the second portion 2B are placed over the open end of the vessel body 4, and the fasteners 20 and 24 are secured by their respective ring 22. As described above, in the lid 2 of this embodiment, the middle portions of the tube 32 and the stirring rod 33 extending vertically can be inserted in the direction parallel to the lid 2 from the insertion slit 18 opened in the outer peripheral surface and the division plane 10 of the lid 2 to push the tube 32 and the stirring rod 33 into the insertion slits 18, and the tube 32 and the stirring rod 33 can be moved along the insertion slits 18 to extend to the circular portion 8B, the circular portion 8D, and the T-shaped portion 8F, and the tube 32 and the stirring rod 33 can be held at arbitrary positions of the insertion slits 18 in the circular portion 8B, the circular portion 8D, and the T-shaped portion 8F. A stirring blade 35 is attached to the lower end of the stirring rod 33, and the stirring rod 33 is rotated about an axis by a motor (illustration is abbreviated) disposed outside the vessel 1. In the vessel 1, the position of the stirring rod 33 can also be fixed at an arbitrary position along the insertion slits 18 of the lid 2 instead of the center of the lid 2 illustrated in the drawing. In addition to the tubes 32 and the stirring rod 33, cables of various sensors, and the like can also be held through the insertion slits 18. Hereinafter, the tube 32, the stirring rod 33, the cable, and the like will be referred to as "tube 32 and the like".

Even in the held state, the tube 32 and the like can be moved up and down and right and left by applying a force, and the holding positions for the tube 32 and the like can be arbitrarily changed according to the contents and the tube arrangement state of the cell culture device. Since the insertion slit 18 is substantially closed at a portion where the tube 32 and the like are not held, the heat does not flow in and out from the insertion slit 18 and the heat insulating property of the lid 2 is not impaired, so that the temperature of the content in the vessel body 4 can be accurately managed.

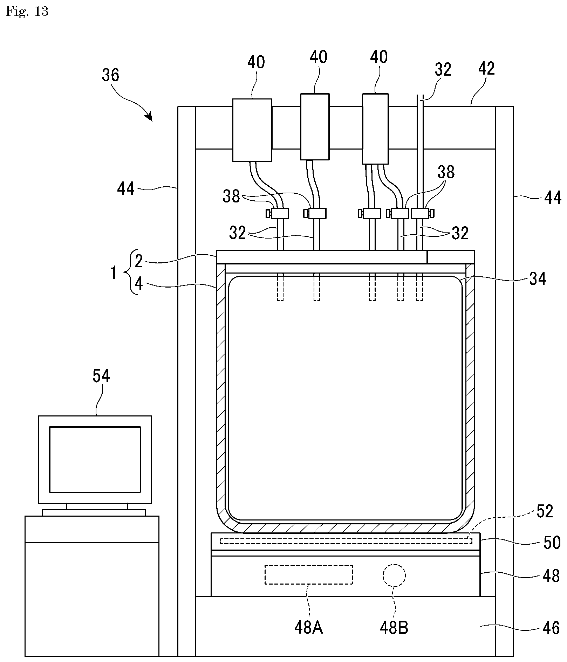

Fig. 13 illustrates a state in which the vessel 1 is set in a cell culture device (culture device) 36. The culture device 36 includes a base 46, a shaking unit 48 disposed on the base 46, a stand 50 that is shaken by the shaking unit 48, and a heater (temperature adjustment mechanism) 52 disposed in the stand 50. The shaking unit 48 includes, for example, actuators 48A and 48B, and the stand 50 is shaken by these actuators 48A and 48B.

Frames 44 are erected from both sides of the base 46, a support portion 42 is fixed to the upper portion of the frames 44, and a plurality of flow rate adjusting units 40 and the like are attached to the support portion 42. Each of the tubes 32 is connected via a valve 38, as necessary, to the tube directly attached to the flow rate adjusting unit 40 or the support portion 42, and is extended into the vessel body 4 through the insertion slit 18 of the lid 2 described above. The lower end position of the tube 32 may be determined as necessary, and may or may not extend to the bottom portion in the culture bag 34. The lower end of the illustrated tube 32 is disposed on the upper portion of inside of the vessel 1. The shaking unit 48 and the heater 52 can control the shaking speed and the temperature by the control portion 54.

A culture bag 34 made of a plastic or the like in which a liquid (culture solution) is sealed is housed in the vessel body 4. However, it is also possible to directly put the culture solution into the vessel body 4. The tubes 32 and the like are inserted in an airtight manner into the upper end portion of the culture bag 34 via an airtight seal (illustration is abbreviated). A liquid containing nutrients and chemicals necessary for culture may be injected, or oxygen gas or carbon dioxide gas may be supplied into the culture bag 34 or discharged from the culture bag 34 to outside through each of the corresponding tubes 32 and the like.

According to the lid 2, the vessel 1, and the culture device 36 configured by the above constitutions, when the tube 32 and the like are fixed through the lid 2, the insertion slit 18 formed in the flexible member 8 can be widened to insert the tube 32 and the like from a side surface, the tube 32 and the like can be moved to an appropriate position along the insertion slit 18, and the tube 32 and the like after the movement can be held by an elastic force of the insertion slit 18. Therefore, excellent effects that the tube 32 and the like can be easily handled and positioned, and that the risk of ingress of foreign matters is low as compared with the case of passing through a hole from an end portion of the tube 32 and the like, are exerted.

Furthermore, since the insertion slit 18 can be widened to insert the tube 32 and the like, and the tube 32 and the like can be moved to an appropriate position in a state where the lid body 6 is divided into two or more with the division plane 10 as a boundary, the tube 32 and the like can be easily handled and positioned.

Furthermore, since the tube 32 and the like can be inserted from a plurality of portions by widening the end portion of the insertion slit 18 that extends to the outer peripheral edge of the lid body 6, and the end portion of the insertion slit 18 that extends to the division plane 10, the tube 32 and the like can be handled and positioned more conveniently.

In addition, since the insertion slit 18 that extends to the outer peripheral edge of the lid body 6 and the plurality of insertion slits 18 branching therefrom are formed in the lid body 6, the tube 32 and the like can be inserted from the insertion slit 18 opening in the outer peripheral edge of the lid body 6, and then the tube 32 and the like can be moved to appropriate positions along the plurality of insertion slits 18 branching from the insertion slit 18. Therefore, the degree of freedom of the holding positions for the tube 32 and the like is high.

Furthermore, since it is possible to move the tube 32 and the like by selecting any of the plurality of insertion slits 18 of the above-mentioned circular portions 8B and 8D and the T-shaped portion 8F after inserting the tube 32 and the like from the side surface along the insertion slit 18 of the linear portion 8A, the degree of freedom of holding positions for the tube 32 and the like is high and thus the tube 32 and the like can be easily handled and positioned.

In addition, since the fasteners 20 and 24 and the ring 22 for fixing the lid 2 and the vessel body 4 to each other are provided as fixing portions on the lid 2 and the vessel body 4, the lid 2 can be fixed in a state of being fitted to the open end of the vessel body 4 by the fixing portion, and the lid 2 can be prevented from coming off from the vessel body 4 even when the vessel 1 is shaken.

In addition, according to this culture device 36, the tube 32 and the like can be easily handled and positioned, and intrusion of foreign matters into the tube 32 and the like can also be prevented, and it is also possible to obtain a heat retaining effect by the lid 2 without inferiority by appropriately shaking the vessel 1 by the shaking unit 48 while adjusting the temperature of the vessel 1 by the heater 52.

### [Second Embodiment]

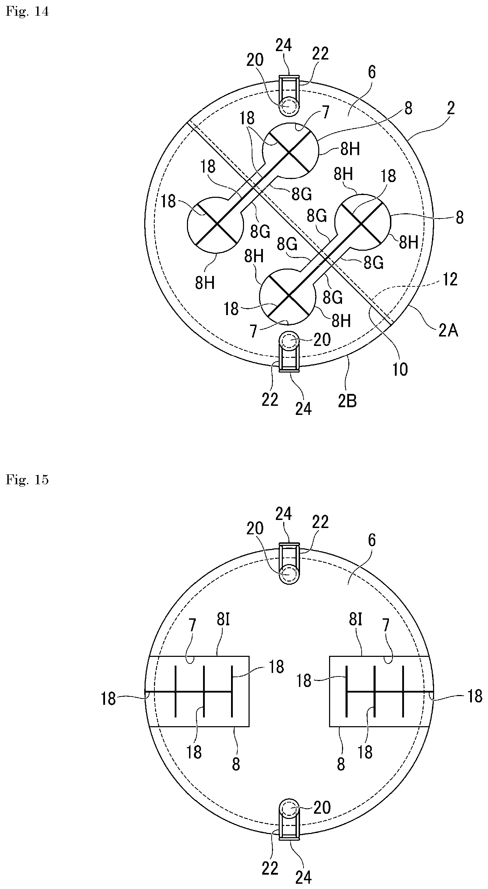

Fig. 14 is a plan view of the lid 2 according to the second embodiment of the present invention, and the components that are common with those of the first embodiment are denoted by identical reference numerals, and descriptions thereof are appropriately omitted. In the second embodiment, two linear portions 8G are formed symmetrically on each of both sides of the division plane 10 with the division plane 10 interposed therebetween, and a total of four circular portions 8H (wide portions) are formed at the other ends of these linear portions 8G. In addition to the insertion slits 18 extending through the linear portions 8G and the circular portions 8H, insertion slits 18 extending in the radial direction of the circular portion 8H are also formed. There is no insertion slit 18 that opens to the outer periphery of the radial lid body 6. In the second embodiment, the tube 32 and the like can be inserted from the insertion slits 18 opened at four positions in the division plane 10, and the tube 32 and the like can be fixed at an arbitrary position of the insertion slits 18 radially formed in the circular portion 8H, and thus the scope of selection of the position to fix the tube 32 and the like is wide. Furthermore, since the insertion slits 18 are not opened in the outer peripheral portion of the lid body 6, there is an advantage that the rigidity of the lid 2 can be easily increased.

### [Third Embodiment]

Fig. 15 is a plan view of the lid 2 according to the third embodiment of the present invention, and the components that are common with those of the first embodiment are denoted by identical reference numerals, and the explanations thereof are appropriately omitted. In the third embodiment, the division plane 10 is not formed on the lid body 6, and thus the lid body 6 is not dividable. Furthermore, two insertion portions 7 are formed in the outer peripheral portion of the lid body 6, rectangular flexible members 8 are fitted into these insertion portions 7 without any gap, and insertion slits 18 that extend to the outer periphery of the lid 2 and a plurality of insertion slits 18 intersecting therewith are formed in the flexible member 8. In the third embodiment, the tube 32 and the like can be inserted from the insertion slits 18 opened at two positions on the outer periphery of the lid 2, and the tube 32 and the like can be fixed at an arbitrary position of the respective six branched insertion slits 18, and thus the scope of selection of the position to fix the tube 32 and the like is wide although there is no division plane 10. In addition, since the division plane 10 is not formed in the lid body 6, there is an advantage that the rigidity of the lid 2 is easily increased.

### [Fourth Embodiment]

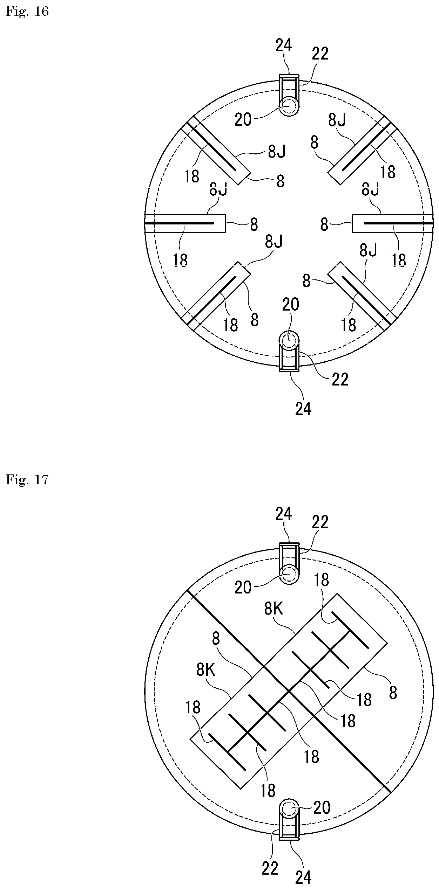

Fig. 16 is a plan view of the lid 2 according to the fourth embodiment of the present invention, and the components that are common with those of the first embodiment are denoted by identical reference numerals, and the explanations thereof are appropriately omitted. In the fourth embodiment, the division plane 10 is not formed in the lid body 6, and the lid body 6 is not dividable, and further, six elongated insertion portions 7 are formed in the outer peripheral portion of the lid body 6, rod-shaped flexible members 8 are fitted into these insertion portions 7 without any gap, and an insertion slit 18 that extends to the outer periphery of the lid 2 is formed in each of the flexible members 8. In the fourth embodiment, the tube 32 and the like can be inserted from the insertion slits 18 opened at six positions in the outer periphery of the lid 2, and the tube 32 and the like can be fixed at an arbitrary position of the insertion slits 18, and thus the scope of selection of the position to fix the tube 32 and the like is wide although there is no division plane 10. In addition, since the division plane 10 is not formed in the lid body 6, there is an advantage that the rigidity of the lid 2 is easily increased.

### [Fifth Embodiment]

Fig. 17 is a plan view of the lid 2 according to the fifth embodiment of the present invention, and the components that are common with those of the first embodiment are denoted by identical reference numerals, and the explanations thereof are appropriately omitted. In the fifth embodiment, the division plane 10 is formed in the lid body 6, two rectangular insertion portions 7 opened to the division plane 10 are formed in the lid body 6, rectangular flexible members 8 are fitted into these insertion portions 7 without any gap, and an insertion slit 18 that extends to the division plane 10 and a plurality of insertion slits 18 intersecting with the insertion slit 18 are formed in each flexible member 8. In the fifth embodiment, the tube 32 and the like can be inserted into the division plane 10 of the lid 2 from the insertion slits 18 opened at two positions, and the tube 32 and the like can be then fixed at arbitrary positions of each of the six branching insertion slits 18. Even though there is no insertion slit 18 opened on the outer periphery, the scope of selection of the position to fix the tube 32 and the like is wide. Furthermore, since any insertion slit 18 opened in the outer periphery of the lid body 6 is not formed, there is an advantage that the rigidity of the outer peripheral portion of the lid 2 is easily increased.

Although the present invention has been described above with reference to a plurality of embodiments, the present invention is not limited only to the above-mentioned embodiments, and it is also possible to sort out and combine the individual components of the above-mentioned embodiments within the scope described in the Claims, and it is also possible to delete some components, add well-known techniques, and the like.

Furthermore, not only the tube 32 but also a thermometer, a supporting rod that supports an object in the vessel body 4, cables of various sensors, and the like may also be inserted into the lid 2.

### INDUSTRIAL APPLICABILITY

According to the lid, the vessel and the cell culture device of the present invention, when the tube and the like are disposed through the above-mentioned lid, the above-mentioned insertion slit can be widened to insert the tube and the like from a side surface, the tube and the like can be moved to an appropriate position along the above-mentioned insertion slit, and the tube and the like after the movement can be held by an elastic force of the above-mentioned insertion slit. Therefore, the tube and the like can be easily handled and positioned, and the risk of ingress of foreign matters from an end portion of the tube and the like is low.

### DESCRIPTION OF THE REFERENCE NUMERALS

1 vessel
2 lid
2A first portion
2B second portion
2C engaging portion
4 vessel body
6 lid body
7 insertion portion
8A, 8C, 8E, 8G, 8J linear portion
8B, 8D, 8H circular portion (wide portion)
8F T-shaped portion (wide portion)
8I, 8K rectangular portion
10 division plane
12, 14, 16 engaging portion
18 insertion slit
20, 24 fastener
22 ring
26 engaging portion
28 slit
30 ridge portion
32 tube
34 culture bag
36 cell culture device
38 valve
40 flow rate adjusting unit
42 support portion
44 frame
46 base
48 shaking unit
48A, 48B actuator
50 stand
52 heater (temperature adjustment mechanism)
54 control portion

## Claims

1. A lid for occluding an open end of a vessel body for a cell culture vessel or a bioreactor,
wherein the lid comprises a lid body having a heat insulation function,
an insertion portion communicating from a front surface to a back surface of the lid body is formed in the lid body, and
the insertion portion is provided with a flexible member having an insertion slit that can be elastically widened.

2. The lid according to claim 1, wherein the lid body is allowed to be divided into two or more parts with a division plane as a boundary.

3. The lid according to claim 1 or 2, wherein the insertion portion extends to an outer peripheral edge of the lid body or the division plane formed in the lid body.

4. The lid according to any one of claims 1 to 3, wherein the insertion portion and the flexible member have an identical shape, and the flexible member is embedded in the insertion portion.

5. The lid according to any one of claims 1 to 4, wherein a first insertion slit that extends to the outer peripheral edge of the lid body and a second insertion slit branching from the first insertion slit are formed as the insertion slit in the insertion portion.

6. The lid according to any one of claims 1 to 5, wherein the insertion portion has a wide portion formed to be wider than other portions of the insertion portion in plan view.

7. The lid according to claim 6, wherein the wide portion has a substantially circular shape in plan view.

8. A vessel for a cell culture device or a bioreactor, comprising:
a vessel body having an open end; and
the lid according to any one of claims 1 to 7.

9. The vessel for a cell culture device or a bioreactor according to claim 8, wherein a fixing portion for fixing the lid and the vessel body to each other is formed on the lid and the vessel body.

10. The vessel for a cell culture device or a bioreactor according to claim 8 or 9, comprising an inner bag in the vessel body.

11. A cell culture device comprising:
the vessel for a cell culture device or a bioreactor according to any one of claims 8 to 10;
a shaking mechanism for shaking the vessel;
a temperature adjustment mechanism for adjusting the temperature of the vessel; and
a tube to be inserted into the vessel through the insertion slit of the lid.
